(19) Europäisches Patentamt
European Patent Office
Office européen des brevets

(11) Publication number: **0 409 499 A2**

(12) # EUROPEAN PATENT APPLICATION

(21) Application number: **90307685.9**

(22) Date of filing: **13.07.90**

(51) Int. Cl.[5]: **A61K 31/495**, C07D 241/08

(30) Priority: **13.07.89 GB 8916072**

(43) Date of publication of application:
**23.01.91 Bulletin 91/04**

(84) Designated Contracting States:
**AT BE CH DE DK ES FR GB GR IT LI LU NL SE**

(71) Applicant: **NATIONAL RESEARCH DEVELOPMENT CORPORATION**
**101 Newington Causeway**
**London SE1 6BU(GB)**

(72) Inventor: **Creighton, Andrew Malcolm**
**68 Millway**
**Mill Hill, London NW7 8QY(GB)**

(74) Representative: **Stephenson, Gerald Frederick**
**Patent Department National Research Development Corporation 101 Newington Causeway**
**London SE1 6BU(GB)**

(54) **Pharmaceutical compositions.**

(57) Compounds of formula II:

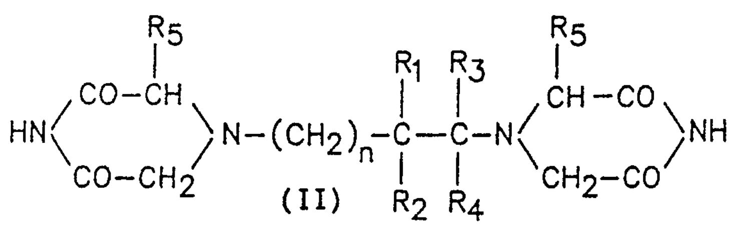

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ as hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, and (b) when n is 0, $R_1$, $R_3$ and $R_5$ are each hydrogen, and each of $R_2$ and $R_4$ is other than hydrogen the compound is in other than the meso or erythro configuration, or a salt thereof formed with a physiologically acceptable inorganic or organic acid, are of use in therapy, particularly as cardioprotective agents.

EP 0 409 499 A2

## PHARMACEUTICAL COMPOSITIONS

This invention relates to pharmaceutical compounds and to compositions containing them, being primarily concerned with substances of use as cardioprotective agents and in certain other protective roles.

Certain bis-dioxopiperazines of formula (I) are cytotoxic and have been used in the treatment of cancer. Thus UK patent 1,234,935 describes the compounds of formula (I) having R = $CH_3$ and $R'$ = $R''$ = H (as the dl , d and l isomers); R = $R'$ = $R''$ = H; R = $R'$ = $CH_3$ and $R''$ = H (as the meso isomer); and R + $R'$ = $-CH_2CH_2-$ and $R''$ = H. Of these the first named compound has proved to be of most value although a further compound of formula (I) having R = $R'$ = H and

$R''$ = 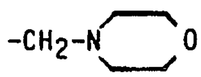

has also been used in treating cancer.

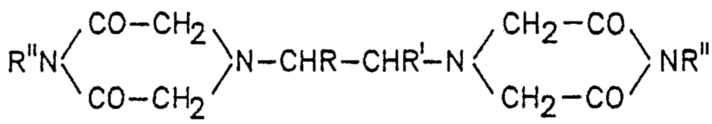

(I)

Studies have been reported by various authors on the chelating properties of these bis-dioxopiperazines and the use in treatment of lead poisoning has been proposed by Wittig and Hultsch, Int. Arch. Occup. Environ. Health, 1981, 48 , 89, for the compound of formula (I) having R = $R'$ = H and $R''$ = $CH_3$ and by May et al , Agents and Actions, 1984, 15 , 448 and Willes and Williams, Plzen. Lek. Sborn., 1985, 49 , 113, for the compound having R = $R''$ = H and $R'$ = $C_2H_5$ in the dl form.

In Research Communications in Chemical Pathology and Pharmacology, 1985, 48 , 39, Herman et al report tests on the protective effect against acute daunorubicin toxicity of a range of bis-dioxopiperazines of formula (I). They conclude that although the compound bimolane (R = $R'$ = H and

$R''$ = 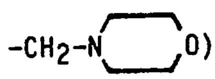)

and the compound having R = $CH_3$ and $R'$ = $R''$ = H (as the dl , d or l isomer) give protection against the lethal effects of daunorubicin, the remainder of the compounds tested (R = $R'$ = $R''$ = H; R = $R'$ = H and $R''$ = $CH_3$; R = $R''$ = $CH_3$ and $R'$ = H ( l ); R = $R'$ = $CH_3$ and $R''$ = H ( meso ); R = $C_2H_5$ and $R'$ = $R''$ = H ( dl ); R = $CH_3$, $R'$ = $C_2H_5$ and $R''$ = H ( dl -erythro ); and R + $R'$ = $-CH_2-CH_2-$ and $R''$ = H; as well as the compound in which $-CHR-CHR'-$ is replaced by $-(CH_2)_3-$ and the ring opened bis-diacid diamide compound having R = $CH_3$ and $R'$ = H) all showed either no protective activity or only minimal protective activity.

It is the case that all of the bisdioxopiperazines identified in this paper as exhibiting a useful level of cardioprotection against daunorubicin toxicity are cytotoxic. We have now found that, despite the indications to the contrary in the paper, various bis-dioxopiperazines which are substantially non-cytotoxic are of interest as cardioprotective agents, as well as in other roles outside the area of lead poisoning. These non-cytotoxic bis-dioxopiperazines are of interest for providing protection against the cardiotoxic effects of various anthracycline drugs but particularly doxorubicin (adriamycin). In this context it is relevant that, in addition to the comments in the Herman et al Research Communications In Chemical Pathology and Pharmacology paper, it is indicated by Herman et al in Advances in Pharmacology and Chemotherapy, 1982, 19 , 249 that even the cardioprotective compound ICRF 159, which is the dl isomer of the compound of formula (I) having R = $CH_3$ and $R'$ = $R''$ = H, is consistently more effective in reducing high dose daunorubicin toxicity than doxorubicin toxicity.

Accordingly the present invention comprises the use of a compound of formula (II):

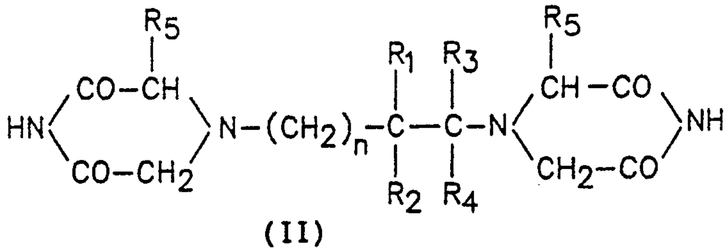

(II)

wherein n is 0, 1 or 2, $R_1$, $R_2$, $R_3$ and $R_4$ are each separately selected from hydrogen, unsubstituted acyclic aliphatic hydrocarbon groups having up to a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, and (b) when n is 0, $R_1$, $R_3$ and $R_5$ are each hydrogen, and each of $R_2$ and $R_4$ is other than hydrogen the compound is in other than the <u>meso</u> or <u>erythro</u> configuration, or salts thereof formed with a physiologically acceptable inorganic or organic acid, for the manufacture of a medicament for use as a cardioprotective agent.

It will be appreciated that the above provisos also extend to equivalent combinations so that, for example, a compound having n = 0, $R_1 = CH_3$ and $R_2 = R_3 = R_4 = R_5 = H$ is the same as that having n = 0, $R_1 = R_2 = R_3 = R_5 = H$ and $R_4 = CH_3$, and the proviso applying to the compounds in which n is 0, $R_1$, $R_3$ and $R_5$ are each hydrogen and each of $R_2$ and $R_4$ is other than hydrogen applies equally to the situation in which n is 0, $R_2$, $R_4$ and $R_5$ are each hydrogen and each of $R_1$ and $R_3$ is other than hydrogen.

In the compounds (II) of the present invention the central grouping in the molecule has the form:

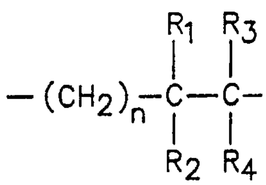

Although n may be 2 or more especially 1, each of $R_1$ to $R_4$ then conveniently being hydrogen, it is preferred that n is 0, in which latter case the grouping will be of the form:

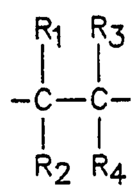

As indicated, $R_1$, $R_2$, $R_3$ and $R_4$ may be hydrogen, an unsubstituted acyclic $C_{1-6}$ aliphatic hydrocarbon group or a $C_{1-6}$ alkyl group substituted by a hydroxy group or a $C_{1-6}$ alkoxy group, subject to the exclusion of two specific compounds in the case when n = 0 which are cytotoxic. The term acyclic aliphatic hydrocarbon group is used herein to include both branched and especially straight chain groups. The group may be unsaturated or especially saturated, conveniently containing one double or triple bond in the former case. Thus, in particular, unsubstituted groups may be alkenyl, alkynyl and particularly alkyl groups, which may be straight or branched chain. The aliphatic hydrocarbon groups conveniently contain a maximum of four or especially three carbon atoms, preferred groups therefore being $C_1$-$C_4$ or $C_1$-$C_3$ alkyl groups and $C_2$-$C_4$ or $C_2$-$C_3$ alkenyl and alkynyl groups.

As regards the substituted $C_{1-6}$ alkyl groups $R_1$ to $R_4$, these may be branched or especially straight chain alkyl groups substituted, particularly terminally, by a hydroxy group or particularly an alkoxy group. Conveniently the alkyl groups are of 1 to 3 or 1 to 4 carbon atoms, substituted ethyl and particularly substituted methyl groups being of most interest. Preferred alkoxy group substituents similarly contain 1 to 3 or 1 to 4 carbon atoms with ethoxy and particularly methoxy groups being of most interest. Conveniently

the total number of carbon atoms in such an alkoxyalkyl group is from 2 to 6, particularly 2 to 4 and especially 2 or 3. Examples of specific substituted groups $R_1$, $R_2$, $R_3$ and $R_4$ are the groups hydroxymethyl, 2-hydroxyethyl and methoxymethyl. Preferably no more than two of $R_1$ to $R_4$, for example $R_1$ and $R_3$ or $R_2$ and $R_4$, and especially only one is a substituted alkyl group and when this is the case the others of $R_1$ to $R_4$ are conveniently hydrogen.

It is generally preferred, however, that where they do not constitute bridged groups the groups $R_1$, $R_2$, $R_3$ and $R_4$ are selected from hydrogen and unsubstituted acyclic aliphatic hydrocarbon groups, especially from the group consisting of hydrogen, methyl, ethyl, n-propyl, isopropyl, allyl and propargyl. Preferably $R_1$ is hydrogen and conveniently either $R_2$ is also hydrogen but $R_3$ and $R_4$ are not or, more usually, $R_3$ is also hydrogen whilst $R_2$ and $R_4$ are either hydrogen or not, for example conveniently being selected from the whole group specified above, but within the proviso given hereinbefore that compounds having n = 0 and $R_1 = R_2 = R_3 = R_4 = R_5$ = H or $R_1 = R_2 = R_3 = R_5$ = H, $R_4 = CH_3$ are excluded. Also of interest, however, are compounds containing a bridging group, particularly a tetramethylene group. Examples of compounds of particular interest are those in which n is 0, $R_1$ and $R_2$ are each hydrogen and $R_3$ and $R_4$ are each methyl, or more particularly n is 0, $R_1$ and $R_3$ are each hydrogen and (a) $R_2$ is hydrogen and $R_4$ is hydrogen or methyl (when $R_5$ is not hydrogen), or ethyl, n-propyl, isopropyl, allyl or propargyl or (b) $R_2$ is methyl or ethyl and $R_4$ is methyl, ethyl, n-propyl, isopropyl, allyl or propargyl.

Specific combinations of particular interest are n = 0, $R_1 = R_2$ = H, $R_3 = R_4 = CH_3$; n = 0, $R_1$ $R_2$ = $R_3$ = H, $R_4 = CH_2OH$ or $CH_2OCH_3$; particularly n = 0, $R_1 = R_3$ = H, $R_2 = R_4 = CH_3$; n = 0, $R_1$ = $R_3$ = H, $R_2 + R_4 = CH_2CH_2CH_2CH_2$; and especially n = 0, $R_1 = R_2 = R_3$ = H, = $C_2H_5$. Additional combinations of interest when $R_5$ is not hydrogen are n = 0, $R_1 = R_2 = R_3 = R_4$ = H and especially n = 0, $R_1 = R_2 = R_3$ = H, $R_4 = CH_3$.

As regards the groups $R_5$ one preference is for these to be hydrogen but where this is not the case the groups may conveniently be a $C_{1-4}$ aliphatic hydrocarbon group, particularly a $C_{1-4}$ or $C_{1-3}$ alkyl group or, to a lesser extent, a $C_{2-4}$ or $C_{2-3}$ alkenyl or alkynyl group; an ethyl or especially a methyl group being of most interest and constituting another preference. When $R_5$ is other than hydrogen each of $R_1$, $R_2$, $R_3$ and $R_4$ is preferably hydrogen or alternatively $R_1$, $R_2$ and $R_3$ are each hydrogen and $R_4$ is ethyl or especially methyl.

Specific preferred compounds according to the present invention are those having the specific combinations of n, $R_1$, $R_2$, $R_3$ and $R_4$ as indicated previously, together with $R_5$ being ethyl, particularly methyl or especially hydrogen. Among these compounds may particularly be mentioned those in which n = 0, $R_1 = R_2 = R_3$ = H, $R_4 = CH_3$, $C_2H_5$ or especially H, $R_5 = CH_3$; n = 0, $R_1 = R_3$ = H, $R_2 = R_4 = CH_3$, $R_5$ = H; and especially n = 0, $R_1 = R_2 = R_3 = R_5$ = H, $R_4 = C_2H_5$. Compounds in which n = 0, $R_1 = R_2 = R_3$ = H, $R_4 = CH_2OH$ and $R_5$ = H or $CH_3$ may have particular value through their enhanced water solubility.

As indicated hereinbefore, when n is 0 and $R_1$, $R_3$ and $R_5$ are each hydrogen but neither $R_2$ nor $R_4$ is hydrogen, the compounds (II) are limited to those in which the groups $R_2$ and $R_4$ are oppositely disposed in the dl or threo configuration (including the enantiomeric forms thereof) rather than adjacently disposed in the meso or erythro configuration, compounds of this latter type being cytotoxic. In other cases where such stereoisomerism can occur the compounds may be in either form. Moreover, compounds in which two of $R_1$ to $R_4$ provide a bridging group may be in the cis or trans form. It will also be appreciated that when the grouping $-(CH_2)_n-CR_1R_2-CR_3R_4-$ does not have a centre of symmetry, the compounds can exist in enantiomorphic d and l forms. In addition, when $R_5$ is not hydrogen this will lead to the presence of two identically substituted asymmetric carbon atoms in the molecule with the possible stereochemical variations this produces. The invention includes the use of the various different isomers of the compounds except where specifically prohibited. In some cases the optically active d - and l -isomers may have the advantage of significantly higher water solubility than the corresponding racemate and it may also be the case that the biological activity of the compound will differ as between the isomers. The invention does therefore extend to the use of such compounds not only as the dl -racemate but also in a form in which the amount of the compound in either the d or l configuration is greater than that in the l or d configuration, respectively (including amounts in that configuration present in the dl racemate). In particular the compound may be essentially in the form of the d or l isomer, for example being substantially free from (i.e. containing no more than 20% and conveniently no more than 10% of) the dl and l or dl and d isomers. However, where the advantage lies in enhanced solubility of the optically active isomers as compared with the racemate, rather than enhanced biological activity for one isomer, any enhancement of the proportion of one isomer should have some effect.

Of the specific compounds previously indicated as being particularly preferred (as the free compound or a salt), 1,2-bis(2-methyl-3,5-dioxopiperazin-1-yl)-ethane, 1,2-bis(2-methyl-3,5-dioxopiperazin-1-yl)-propane, 1,2-bis(2-methyl-3,5-dioxopiperazin-1-yl)-butane, *threo* -2,3-bis(3,5-dioxopiperazin-1-yl)-butane and 1,2-bis(3,5-dioxopiperazin-1-yl)-butane can exist in optically active forms. Among these compounds *dl* -, *d* - and *l* -1,2-bis(3,5-dioxopiperazin-1-yl)-butane are all of especial interest.

It will be appreciated that certain of the compounds of formula (II) as defined hereinbefore are novel and that the present Invention extends to such compounds *per se* , for example *d* - and *l* -1,2-bis(3,5-dioxopiperazin-1-yl)-butane.

Several methods are available for the preparation of the compounds of formula (II). Thus, for example, they can be prepared by reaction of the corresponding tetra-acetic acid of formula (III) with formamide, this reaction usually being carrried out in an excess of formamide as the solvent and at an elevated temperature, preferably under nitrogen.

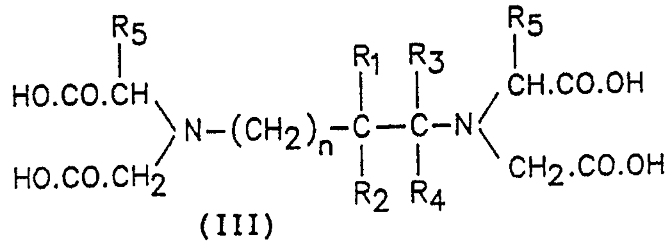

(III)

Alternatively, and with particular advantage when the tetra-acetic acid (III) has a tendency to decarboxylate on heating, the compounds (II) can be prepared by heating the corresponding tetra-amide in polyphosphoric acid or phenol.

A further alternative also exists, having the advantage of being a lower temperature method, which comprises treating the corresponding tetranitrile with sodamide in formamide and treating the resulting product with hydrogen chloride in methanol.

The tetra-acetic acids of formula (III) may conveniently be obtained from the corresponding dicarboxy acid of formula (IV) and the tetra-amides from the corresponding tetra-acetic acid.

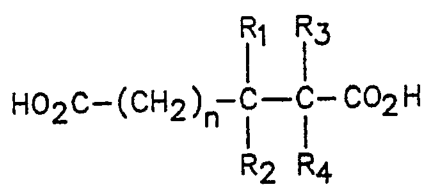

(IV)

Examples of such procedures are to be found in UK patents 961,065, 966,802, 978,724, 1,234,935 and 1,374,979. Intermediates of formula (III) in which $R_5$ is other than hydrogen are described in UK patent 723,316.

In general terms, a process for the preparation of a compound of formula (II) as defined hereinbefore and salts thereof formed with a physiologically acceptable inorganic or organic acid from a compound of the formula (V)

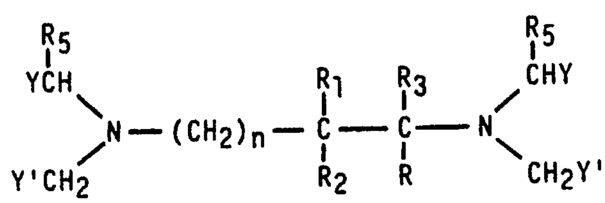

(V)

in which in each instance n has the same value as in the compound of formula (II) comprises:

(a) heating an N,N,N′,N′-tetracarboxymethyl diamine of formula (V) in which $R_1$ to $R_5$ represent groups as in the compound of formula (II) and Y and Y′ represent carboxy groups, or the bis anhydride thereof

wherein $Y$ and $Y'$ together represent a -CO.O.CO-group, with a reagent selected from:

(i) an acid amide of the general formula $RCONH_2$ in which R represents hydrogen, an aliphatic residue, for example an alkyl group, having one to four carbon atoms or an amino group $NH_2$, and

(ii) ammonia in an acid amide of the general formula

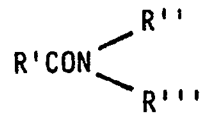

in which $R'$ represents hydrogen or an aliphatic radical, for example an alkyl group, having one to four carbon atoms and $R''$ and $R'''$ represent the same or a different aliphatic radical, for example an alkyl group, having one to four carbon atoms or alternatively one of $R''$ and $R'''$ may with $R'$ and the

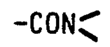

group represent a five or six membered lactam ring;

(b) heating an N,N,N′,N′-tetracarbamoylmethyl diamine of formula (V) in which $R_1$ to $R_5$ represent groups as in the compound of formula (II) and $Y$ and $Y'$ represent carbamoyl groups, with an acidic reagent, such as polyphosphoric acid, phenol or boron trifluoride;

(c) reacting an N,N,N′,N′-tetracyanomethyl diamine of formula (V) in which $R_1$ to $R_5$ represent groups as in the compound of formula (II) and $Y$ and $Y'$ represent cyano groups, with an alkali metal amide and treating the product with acid, for example with a mineral acid;

(d) reacting an N,N′-disubstituted 3,5,3′,5′-tetraoxodipiperazine of formula (V) in which $R_1$ to $R_5$ represent groups as In the compound of formula (II) and $Y$ and $Y'$ together represent a grouping -CO.NX.CO- wherein X is a group convertible to hydrogen, to replace the group X by hydrogen; or, to a lesser extent,

(e) reacting an N,N′-dicarboxymethyl-N,N′-dicarbamoylmethyl diamine of formula (V) in which $R_1$ to $R_5$ represent groups as in the compound of formula (II), one of $Y$ and $Y'$ represents a carboxy group and the other represents a carbamoyl group, to effect cyclisation thereof; and, where appropriate, separating the required compound (II) from other isomers thereof and/or converting the compound (II) to a physiologically acceptable addition salt thereof.

In (a) to (e) above, terms such as "carboxymethyl" include a group $-CH(R_5)-CO_2H$, etc. The preferred procedures are (a), (b) and (c), particularly as described hereinbefore.

To obtain compounds (II) of the desired stereochemistry it is most convenient to use an intermediate compound having the equivalent stereochemistry, particularly as regards the preparation of geometrical isomers, for example compounds in the dl or threo form as opposed to the meso or erythro form, but conveniently also as regards the preparation of optically active compounds. Thus the tetra-acetic acid, tetra-amide, tetranitrile, etc., which is used conveniently has the same stereochemistry as is desired in the final product. When a d or l isomer is required rather than the dl racemate, however, an alternative to the preferred utilisation of a d or l intermediate compound is to effect a resolution of the compound (II), for example using an appropriate optically active acid to form a mixture of salts of the d and l forms of the compound (II) which are then separated.

It will be appreciated from the foregoing discussion that of the compounds (II) only the compound dl -1,2-bis(3,5-dioxopiperazin-1-yl)-butane has previously been indicated in the literature as being of use in therapy. Accordingly the present invention further comprises a compound of formula (II) as defined hereinbefore, but excluding the compound dl -1,2-bis(3,5-dioxopiperazin-1-yl)-butane, for use in therapy.

The present invention also includes pharmaceutical compositions comprising as an active component a compound of formula (II) as defined hereinbefore, but excluding the compounds 1,3-bis(3,5-dioxopiperazin-1-yl)-propane, dl -1,2-bis(3,5-dioxopiperazin-1-yl)butane and their salts, together with a physiologically acceptable diluent or carrier excluding any liquid which is not sterile and pyrogen-free.

As indicated, the compounds (II) may be formulated as salts formed with physiologically acceptable inorganic or organic acids and, when so formulated, it is preferred to use methane sulphonic acid, isethionic acid, tartaric acid or another solubilising acid.

The compounds of formula (II) may be formulated singly, or as a mixture of two or more compounds, for use as pharmaceuticals by a variety of methods. For instance, they may be applied as aqueous, oily (e.g. as a suspension in isopropyl myristate), or in some cases emulsified compositions for parenteral

administration and therefore preferably sterile and pyrogen-free. Some of these compounds have rather low solubility in aqueous media and are therefore usually administered in the form of aqueous suspensions containing suitable surface active agents. It will be appreciated that the dosage levels used may vary over quite a wide range especially since certain of the compounds (II) are more active than others. However, without commitment to a rigid definition of dosages it may be stated that a daily dosage of active constituent (estimated as the free base), divided if necessary, of from 10 mg to 3 g is proposed for parenteral mammalian use. This dosage may conveniently be applied as a solution in 500-1000 ml of liquid for intravenous injection by slow infusion, or as a solution or suspension in about 10 ml of liquid by the intramuscular route, or in small volumes subcutaneously. (Parenteral, particularly intravenous, administration is the route preferred for use in conjunction with the anthracycline drugs so that injectable compositions are of especial interest.) More particularly, with many compounds (II) the daily dose for a 70 kg human, administered parenterally, will often be in the range from about 100 mg to about 500 mg but with the more active compounds it may be less than this (the dose being varied pro rata for humans of a different weight or other mammals). When used in conjunction with an anthracycline drug, where a single administration of the drug and the compound (II) is common, however, higher doses than this may often be employed, for example between about 500 mg and about 3 g with doses of more than this being considered where appropriate in terms of the ratios of compound (II):anthracycline drug as discussed hereinafter.

Where appropriate, the substances may also be compounded for oral administration in dosages which may be similar but may often be somewhat higher, for example in a range from 100 mg to 1 g or even as high as 3 g for the daily dose for a 70 kg human for many compounds (II) but possibly somewhat less than this for the more active compounds. Such oral formulations may particularly take the form of tablets compounded in the presence of conventional solid carrier materials such as starch, lactose, dextrin and magnesium stearate, or of capsules or cachets. Suppositories, pessaries, aerosol and other formulations may also be employed. The compounds may be formulated in unit dosage form, i.e. in discrete portions each containing a unit dose, or a multiple or sub-multiple of a unit dose of the active ingredient.

The compounds (II) as defined hereinbefore are primarily of value as cardioprotective agents and it should be noted that their potential in such a use extends not only to use in conjunction with drugs having a cardiotoxic side effect, these often being cytotoxic agents such as the anthracycline drugs, which are of particular value in treating breast cancer, but also extends to pathological conditions where the heart is at risk. The term "anthracycline drug" is used herein to include not only natural and semi-synthetic anthracyclines such as epirubicin, idarubicin, daunorubicin and especially doxorubicin (which names are used herein to include salts of these compounds), but also synthetic anthracyclines such as mitoxantrone. Indeed, the compounds (II) are of value in providing cardioprotection against the cardiotoxic side effect of various compounds containing a moiety

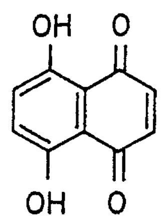

the toxic effect of such compounds being believed to derive from their chelating ability.

The compounds (II) also find a secondary use in protection against other toxic effects arising from natural diseases or induction by drugs, for example by various agents which are either toxic as such or when present in the body in excess, such agents including paracetamol (p-hydroxyacetanilide).

The compounds (II) find most application in the treatment of humans although they can find veterinary use in certain other mammals such as dogs, rabbits, cattle, and horses.

When used as a cardioprotective agent in the context of a pathological condition where the heart is at risk as a result of that condition the compounds (II) are administered for a period dictated by the existence of this condition. When used in a cardioprotective role in conjunction with a drug having a cardiotoxic side effect, the period of administration will be related to that of the use of the drug which will usually be administered at normal dosage rates and by the usual regimen, often parenterally. The compounds (II) may conveniently be administered before, together with or less often after the drug, the choice depending to some extent on the particular drug in question. In the first and third usages both the compound (II) and the drug will each be formulated separately, usually in a conventional manner, for example both being formulated as described above, although the two compositions may be packaged together for ease of

sequential administration to the patient. A suitable time lapse between administration of the compound (II) and the drug in either order is quite short, being no more than about 1 to 4 hours, for example 2 hours, and particularly being about 1 hour or somewhat less, depending on the drug in question.

When the compound (II) is administered together with the drug, the two may be formulated separately but it may be preferred to include the compound (II) and the drug in the same composition. Such a pharmaceutical composition may again conveniently take one of the forms described above for compositions containing only the compound (II) and may, if desired, contain more than one compound (II) and/or more than one drug. The present invention thus includes (a) a pharmaceutical composition which comprises a compound of formula (II), as defined hereinbefore, and a drug having a cardiotoxic or other toxic side effect, for example an anthracycline drug, together with a physiologically acceptable diluent or carrier, and also (b) a kit comprising in association a compound of formula (II), as defined hereinbefore, and a drug having cardiotoxic or other toxic side effect.

As indicated, the compounds (II) are of particular interest for use with doxorubicin and the present Invention therefore particularly includes a pharmaceutical composition comprising a compound of formula (II) as defined hereinbefore, for example dl -, d - or l -1,2-bis(3,5-dioxopiperazln-1-yl)butane or a salt thereof, and doxorubicin, together with a physiologically acceptable diluent or carrier.

In instances where a series of doses of the drug is administered it may not be necessary for each administration of the drug to be made concomitantly with, or at the interval given above after or before the administration of the compound (II). It may be possible to administer the compound (II) alone or together with the drug, followed by one or more repeated spaced doses of the drug alone or, more often, in view of the more rapid metabolisation of the compound (II), to administer the drug alone or together with the compound (II), followed by one or more repeated spaced doses of the compound (II) alone. If the treatment with the drug is continued over an extended period repeat doses of the compound (II) are also likely to be required and one possible regimen would involve the administration of the drug and compound (II) together on certain occasions followed by the compound (II) alone on others.

As regards the relative amounts of the compound (II) and a drug to be used, this will depend on both the particular compound (II), the drug used and the regimen of use, a good indication being provided, however, by the dosages indicated hereinbefore for the compounds (II) and the conventional doses used for the drug. However, some additional comments may be made concerning the proportions of compound or compounds (II) to anthracycline drug which are used either singly or together in a pharmaceutical composition containing both a compound or compounds (II) and an anthracycline drug. Thus, by way of guidance it may be stated that a dose ratio of between 5:1 to 20:1 or even 25:1 w/w of compound or compounds (II) to drug, especially about 10:1 w/w, is often suitable. By way of further guidance, it may be mentioned that a normal single dosage of doxorubicin is in the range of about 0.75 to 2 mg/kg, i.e. about 50 to 150 mg for a 70 kg human being, but that the use of the compounds (II) is intended to enable some increase in the dosage, for example to 4 or 5 mg/kg, if desired, in order to enhance the anti-cancer effect of the doxorubicin whilst its cardiotoxic side effects are controlled by the presence of the compound (II).

The exact dosage of an anthracycline drug such as doxorubicin which is used will depend on whether it is given with other anti-tumour agents. Thus anthracycline drugs are often given together with one or more of other such agents, for example fluorouracil and cyclophosphamide and, where desired, a pharmaceutical composition containing a compound or compounds (II) and an anthracycline drug can contain other such anti-tumour agents. Moreover, it may be advantageous to administer a calcium supplement together wih the compounds (II), this usually being administered separately.

When used as a protective agent against the toxic effect of paracetamol, the compounds (II) may be used protectively before occurrence of the toxicity or following occurrence of the toxicity. It may even be possible to formulate the compound (II) with paracetamol in order automatically to counter the effect of an overdose thereof. Broadly similar dosage levels may be used to those described hereinbefore although where the toxic effect is acute, as for example is usually the case following an overdose of paracetamol, higher dosages over a shorter period may be indicated.

Other forms of protection include the use of the compounds (II) in conjunction with any condition which is either "naturally occurring" or drug induced where damage caused by free radicals occurs (this may also be involved in some of the conditions described hereinbefore such as anthracycline drug-induced damage), for example in reducing the diabetogenic effect of drugs such as alloxan which generate free hydroxyl radicals. The compounds (II) may once again be used in a broadly similar manner as when employed in cardioprotection, including formulation together with the drug, and the dosage levels used.

The present invention thus includes a method of providing protection against a toxic effect on the body, particularly a cardiotoxic effect, which comprises administering to a patient in need thereof a therapeutically effective amount of a compound (II) as defined hereinbefore.

The present invention is illustrated by the following Examples.

## EXAMPLES

### Example 1 : Preparation of dl-1,2-bis(3,5-dioxopiperazin-1-yl)-butane

dl -1,2-Diaminobutane tetra-acetic acid (2.0 g) (prepared essentially as described by Novak et al , Chem. Zvesti, 1968, 22 , 723) and formamide (40 ml) are heated together under reduced pressure in an atmosphere of nitrogen at 100-110 °C for 1 hour and then at 155 °C for 4 hours. The resulting solution is evaporated under reduced pressure to give a semi-solid residue which is crystallised from methanol to give dl -1,2-bis(3,5-dioxopiperazin-1-yl)-butane (0.97 g, 57%), m.p. 219-221 °C, $\nu_{max}$ (nujol) 1700, 3050, 3170 $cm^{-1}$.

### Example 2 : Preparation of (S)-1,2-bis(3,5-dioxopiperazin-1-yl)butane

(S)-2-aminobutyric acid (10 g) is converted into the methyl ester hydrochloride, m.p. 100-103 °C, using thionylchloride/methanol according to the method of Mazur et al , J. Med. Chem., 1973, 16 , 1284. A solution of the free base of the ester in methanol is saturated with ammonia at 0 °C and retained in a stoppered flask until t.l.c. indicates that the reaction is complete (about 7 days). The solvent is then evaporated to give an oil which is crystallised on cooling to give (S)-2-aminobutyramide (7.2 g, 91%), m.p. 64-65 °C, from isopropanol/ether. The amide (7.1 g) in tetrahydrofuran (100 ml) is cooled with ice while 500 ml of 1M borane-THF is added under nitrogen over 2 hours. The mixture is heated under reflux overnight and cooled and stirred during the addition of methanol (100 ml) to destroy the excess borane. The mixture is evaporated to about 100 ml. The residue is diluted with ethanol (100 ml) and cooled and stirred during the addition of 45% HBr/acetic acid (100 ml). The reaction mixture is heated under reflux overnight, washed and filtered and then re-crystallised from ethanol/ether to give (S)-1,2-diaminobutane dihydrobromide (11.7 g, 66%), m.p. 221-224 °C (compared to 132-135 °C for the dihydrobromide of the corresponding racemate).

The diamine salt (10.5 g) is carboxymethylated by the method of Okaku et al , Bull. Soc. Chem. Japan, 1967, 40 , 2326, and the product is isolated by passage through an AG50 WX8 cation exchange resin (280 ml settled resin in $H^+$ form). After the elution of halide-containing non-chelating material with water, (S)-1,2-diaminobutane tetra-acetic acid (5.5 g, 41%), m.p. 217-220 °C, is obtained by crystallisation from the subsequent eluate. Treatment of the tetra-acetic acid with formamide at 155 °C for 5 hours gives (5)-1,2-bis-(3,5-dioxopiperazin-1-yl)-butane (40%), m.p. 177-178 °C., from methanol/acetone (compared to 219-221 °C for the corresponding racemate).

### Example 3 : Preparation of 1,2-bis(3,5-dioxopiperazin-1-yl)-2-methylpropane

2-Methyl-1,2-diaminopropane tetra-acetic acid and an excess of formamide (1:5 to 1:20 w/v of tetra-acetic acid:formamide) are heated together under reduced pressure in an atmosphere of nitrogen at 100-110 °C for 1 hour and then at 155-160 °C for 5 hours. The resulting solution is evaporated to dryness and the residue is crystallised from methanol/acetone to give 2-methyl-1,2-bis(3,5-dioxopiperazln-1-yl)-propane in 10% yield, m.p. 206 °C, $\nu_{max}$ (nujol) 1660 (shoulder), 1690, 1740, 3050, 3170 $cm^{-1}$.

### Example 4 : Preparation of 1,2-bis(2-methyl-3,5-dioxopiperazin-1-yl)-ethane

1,2-Diaminoethane-N,N′-diacetic acid-N,N′-di-2-propionic acid (prepared from alanine and ethylene dibromide essentially as described in UK Patent 723,316) and an excess of formamide (1:5 to 1:20 w/v of diacetic acid di-2-propionic acid:formamide) are heated together under reduced pressure in an atmosphere of nitrogen at 100/110 °C for 1 hour and then at 155-160 °C for 5 hours. The resulting solution is evaporated to dryness and the residue is extracted with dioxan. Concentration of the dioxan solution gives 1,2-bis(2-methyl-3,5-dioxopiperazin-1-yl)-ethane in 5% yield (based on the ethylene bromide used to prepare the diacetic acid di-2-propionic acid), m.p. 231-232 °C from dioxan/cyclohexane, $\nu_{max}$ (nujol) 1690, 1730, 3050,

3200 $cm^{-1}$.

Example 5 : Preparation of 1,3-bis(3,5-dioxopiperazin-1-yl)-propane

1,3-Diaminopropane tetra-acetic acid (34.4 9) and formamide (150 ml) are heated together under reduced pressure in an atmosphere of nitrogen at 100-110 °C for 1.5 hours and then at 155 °C for 4.5 hours. The solution is evaporated to dryness and the resultant residue is recrystallised from dimethyl formamide/methyl ethyl ketone to give 1,3-bis(3,5-dioxopiperazin-1-yl)-propane (13.8 g, 46%), m.p. 204-205 °C, $\nu_{max}$ (nujol) 1700, 1725 (shoulder), 3150 (shoulder) $cm^{-1}$.

Example 6 : Preparation of dl-2,3-bis(3,5-dioxopiperazin-1-yl)-butane

dl -2,3-Diaminobutane tetraacetic acid monohydrate (5.7 g) (prepared essentially as described by Irving and Parkins, J. Inorg. Nucl. Chem., 1966, 2 8, 1629) is heated with formamide (40 ml) under nitrogen at about 155 °C for 5 hours. The reaction mixture is then cooled and evaporated to dryness, and the residue is triturated with methanol. The crude product is recrystallised from dimethylformamide/methyl ethyl ketone to give dl -2,3-bis(3,5-dioxopiperazin-1-yl)-butane (1.1 g, 23%), m.p. 267-269 °C (dec.).

Example 7 : Preparation of dl-threo-2,3-bis(3,5-dioxopiperazin-1-yl)-pentane

2,3-Dihydroxyiminopentane (70 g) in ethanol (200 ml) is hydrogenated over Raney Nickel catalyst (25 ml settled slurry, Type 102) for 48 hours at atmospheric pressure and 20 °C, by which time the uptake of hydrogen should be complete. Distillation of the filtered solution affords a mixture of the diastereoisomers of 2,3-diaminopentane (36 g, 51%), b.p. 60-70 °C/50 mm.

A solution of benzyl chloroformate (43 ml) in dioxan (125 ml) is added to a suspension of the diamine isomers (12 g) in water (70 ml) with stirring over 1 hour. The mixture is cooled during the reaction to maintain the temperature at 20-25 °C and the pH is maintained at about 9.5 by the periodic addition of 6N sodium hydroxide. The residue obtained on evaporation of the reaction mixture is dissolved in dichloromethane (100 ml) and washed with water (2 x 100 ml). Evaporation of the dried ($Na_2SO_4$) solution gives a crystalline residue containing a mixture of the crude dl -erythro and dl -threo isomers of 2,3-dibenzyloxycarbonylaminopentane.

Recrystallisation from ethanol yields the dl -erythro isomer (7.2 g, 19.5%), m.p. 196 °C, $\nu_{max}$ l685s, 1325m, 1100w and 905w (w indicates a weak maximum), Rf 0.6 on Kieselgel 60 F254 thin layer chromatography (t.l.c.) eluting with 3:1 v/v chloroform:ethyl acetate. The mother liquors containing the more soluble dl -threo isomer are evaporated to low bulk and chromatographed on a silica gel (Merck grade 60) column eluted with 4:1 v/v chloroform:ethyl acetate. Fractions are collected and monitored by t.l.c. as described above. Appropriate fractions containing the dl -threo isomer with an Rf of 0.7 are combined and evaporated to yield dl -threo -2,3-dibenzyloxycarbonylaminopentane (2.0 g, 5.5%), m.p. 90-91 °C from toluene, $\nu_{max}$ 1695s, 1335w, 1320w, 1295w, 1120w, 915w and 705w.

dl -threo -2,3-Dibenzyloxycarbonylaminopentane (3 g) is added to HBr in acetic acid (24 ml, 45%) with stirring over 30 minutes. The product is precipitated as an oil by the addition of ether (90 ml) and is washed with fresh ether (2 x 50 ml). The resultant crude dl -threo -2,3-diaminopentane dihyrobromide (2.1 g) obtained as a hygroscopic gum is carboxymethylated directly with bromoacetic acid (5.5 g) by the method of Okaku et al , ibid . The tetra-acetic acid is isolated from the reaction miture using cation exchange chromatography (Zeocarb 225, $H^+$ form), the column (250 ml wet resin) being eluted at 60 °C with water. Fractions of low pH which are also free of halide and contain the tetra-acetic acid chelating agent, as shown by a spot test with nickel/dimethylglyoxime), are combined and evaporated to yield dl -threo -2,3-diaminopentane tetra-acetic acid (1.06 g, 38%) as the monohydrate, m.p. 190-195 °C from water.

dl -threo -2,3-Diaminopentane tetra-acetic acid (0.10 g) is heated with formamide under nitrogen at 150-160 °C for 5 hours. The reaction mixture is then cooled and evaporated to dryness, and the residue is triturated with methanol. The crude product is recrystallised from dimethylsulphoxide/methanol to give dl -threo -2,3-bis(3,5-dioxopiperazin-1-yl)-pentane (0.05 g, 56%), m.p. 180 °C, $\nu_{max}$ 3040w, 1695s, 1335w, 1320w, 1295w, 1270w and 1035w.

Example 8 : Preparation of dl-threo-2,3-bis(3,5-dioxopiperazin-1-yl)-1-methoxybutane

1-Methoxy-2,3-dioximinobutane (8.25 g) (prepared from methyl vinyl ketone via 1-methoxybutan-3-one by standard procedures, m.p. 145-146° C) is hydrogenated over Raney Nickel catalyst (10 ml settled slurry, Type 102), activated with chloroplatinic acid (1 ml, 5% w/v), in ethanol (200 ml). The catalyst is removed by filtration and the 2,3-diamino-1-methoxybutane is isolated as the neutral sulphate (2.75 g), m.p. 282-284° C, in the form of a product containing both the erythro and threo isomers.

The diamine sulphate (2.0 g) is carboxymethylated with bromoacetic acid (6.95 g) by the method of Okaku et al , ibid and the product is isolated by passage through a 120 g Zeo-Carb 225 resin (SRC 15) column. The column is eluted with cold water, 10 ml fractions being collected. After elution of halide-containing, non-chelating material in the first 15 fractions dl -erythro -2,3-dlamino-1-methoxybutane tetra-acetic acid is recovered as a semi-crystalline glass by concentrating fractions 39-82 and the corresponding threo isomer is obtained by concentrating fractions 83-116 as a crystalline solid (1.6 g, 63%), m.p. 225-226° C (dec.).

Heating dl -threo -2,3-diamino-1-methoxybutane tetra-acetic acid (500 mg) in formamide (25 ml) at 150-155° C for 5 hours under nitrogen led to the isolation of dl -threo -2,3-bis(3,5-dioxopiperazin-1-yl)-1-methoxybutane (175 mg, 40%), m.p. 222-224° C, from dimethylformamide/methanol. Thin layer chromatography (Kiesilgel 60-Merck, eluting with 10% v/v ethyl acetate/acetone) gives an Rf of 0.45 for this threo isomer, clearly distinct and free from the corresponding erythro isomer which has an Rf of 0.65.

Example 9 : Preparation of dl-trans-1,2-bis)3,5-dioxopiperazin-1-yl)-cyclohexane

dl -trans -1,2-Diaminocyclohexane tetra-acetic acid (21.9 g) and formamide (71 ml) are heated together at 150-160° C with stirring under nitrogen for 5 hours. The acid dissolves quite rapidly but after about $\frac{1}{2}$ hour a fine precipitate begins to separate. At the end of the 5 hours, the reaction mixture is cooled and filtered and the product is washed with water and dried to give analytically pure dl -trans -1,2-bis(3,5-dioxopiperazin-1-yl)-cyclohexane (12.5 g, 67.5%), m.p. 340-341° C (dec).

Example 10 : Formulation of compounds

(A) Tablets of the following composition are prepared:

| | mg/tablet |
|---|---|
| Compound of Example 1 (micronised) | 250 |
| 'Avicel' (microcrystalline cellulose)* | 38 |
| polyvinylpyrrolidone | 3 |
| alginic acid | 6 |
| magnesium stearate | 3 |

* 'Avicel' is a Registered Trade Mark or Service Mark.

The compound of Example 1 is mixed with 'Avicel' and polyvinylpyrrolidone is added, dissolved in sufficient industrial methylated spirits (74° OP) to produce a mass suitable for granulating. The mass is granulated through a 20 mesh sieve and the resultant granules are dried at a temperature not exceeding 50° C. The dried granules are passed through a 20 mesh sieve and the alginic acid and magnesium stearate are then added and mixed with the granules. The product is compressed into tablets each weighing 300 mg on 3/8 inch flat bevelled edge divided punches.

(B) Tablets of the following composition are prepared:

| | mg/tablet |
|---|---|
| Compound of Example 1 (micronised) | 250 |
| 'Avicel' (microcrystalline cellulose) | 134 |
| polyvinylpyrrolidone | 4 |
| alginic acid | 8 |
| magnesium stearate | 4 |

The tablets are prepared by essentially the same procedure as described in (A) and are compressed at a tablet weight of 400 mg on 7/16 inch flat bevelled edge punches.

(C) Tablets of the following composition are prepared:

| | mg/tablet |
|---|---|
| Compound of Example 1 (micronised) | 250 |
| lactose (300 mesh) | 19 |
| maize starch | 15· |
| gelatine | 10 |
| magnesium stearate | 6 |

The tablets are prepared by mixing the compound of Example 1 with lactose and half the total quantity of maize starch required, and adding to the mass a 5% solution of gelatine in water. The product is granulated through a 16 mesh sieve, and the resultant granules are dried to constant weight at a temperature not exceeding 50°C. The dried granules are passed through a 20 mesh sieve and mixed with magnesium stearate and the remainder of the maize starch. The product is compressed at a 300 mg tablet weight on 3/8 inch flat bevelled edge divided punches.

Similar procedures may be followed with the compounds of Examples 2 to 9.

Example 11 : Cardioprotective effect of the compounds (II) against the toxicity of doxorubicin

The following compounds were selected to be tested for their cardioprotetive effect against the toxicity of doxorubicin. The compounds tested were:
1,3-bis(3,5-dioxopiperazin-1-yl)-propane
1,2-bis(2-methyl-3,5-dioxopiperazin-1-yl)-ethane
1,2-bis(3,5-dioxopiperazin-1-yl)-butane.

Groups of growing rats were injected intra-peritoneally (i.p.) with 100 mg/kg or 400 mg/kg body weight of the compound. After 1 hour, doxorubicin solution was injected intravenously (i.v.) at a concentration of 4 mg/kg body weight. Other groups of rats were injected with saline (i.p.) followed after 1 hour by saline (i.v.) or with saline (i.p.) followed after 1 hour by doxorubicin solution (i.v.) as controls. The cardiotoxicity of doxorubicin and the protection supplied by the compounds was assessed by measuring the heart rate and cardiac output relative to the saline + saline control. Cardiac output was measured by injecting $^{99m}Tc$ into the heart and measuring the rate at which the isotope was cleared from the heart. Heart rate was measured in the standard manner.

The results are shown in the Table below from which it will be seen that each of the compounds results in an improvement in cardiac output and heart rate as compared with the depressed values which occur when only doxorubicin is used.

TABLE

| Treatment | Dose of Compound (II) | Relative Cardiac Output | Relative Heart Rate |
|---|---|---|---|
| saline + saline | - | 1.00 ± 0.05 | 1.00 ± 0.02 |
| saline + doxorubicin | - | 0.41 ± 0.04 | 0.65 ± 0.19 |
| 1,3-bis(3,5-dioxopiperazin-1-yl)-propane + doxorubicin | 100 mg/kg | 0.56 ± 0.06 | 0.81 ± 0.12 |
| 1,2-bis(2-methyl-3,5-dioxopiperazin-1-yl)-ethane + doxorubicin | 100 mg/kg | 0.62 ± 0.05 | 0.99 ± 0.03 |
| 1,2-bis(3,5-dioxopiperazin-1-yl)-butane + doxorubicin | 100 mg/kg | 0.70 ± 0.05 | 0.98 ± 0.03 |
| ditto | 400 mg/kg | 0.67 ± 0.05 | 0.92 ± 0.04 |

## Claims

1. The use of a compound of formula (II):

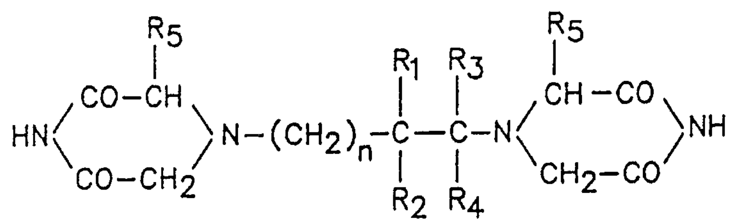

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, and (b) when n is 0, $R_1$, $R_3$ and $R_5$ are each hydrogen, and each of $R_2$ and $R_4$ is other than hydrogen the compound is in other than the <u>meso</u> or <u>erythro</u> configuration, or a salt thereof formed with a physiologically acceptable inorganic or organic acid, for the manufacture of a medicament for use as a cardioprotective agent.

2. The use according to Claim 1, in which $R_1$, $R_2$, $R_3$ and $R_4$ are each separately selected from hydrogen and $C_{1-4}$ alkyl, $C_{2-4}$ alkenyl and $C_{2-4}$ alkynyl groups or one of $R_1$ and $R_2$ and one of $R_3$ and $R_4$ is hydrogen and the others are a trimethylene, tetramethylene or pentamethylene bridging group.

3. The use according to Claim 2, in which $R_1$, $R_2$, $R_3$ and $R_4$ are each separately selected from hydrogen, methyl, ethyl, n-propyl, isopropyl, allyl and propargyl or one of $R_1$ and $R_2$ and one of $R_3$ and $R_4$ is hydrogen and the others are tetramethylene.

4. The use according to any of Claims 1 to 3, in which n is 0.

5. The use according to Claim 1, in which n is 0, $R_1$ and $R_2$ are each hydrogen and $R_3$ and $R_4$ are each methyl, or n is 0, $R_1$ and $R_3$ are each hydrogen and (a) $R_2$ is hydrogen and $R_4$ is ethyl, n-propyl, isopropyl, allyl or propargyl or (b) $R_2$ is methyl or ethyl and $R_4$ is methyl, ethyl, n-propyl, isopropyl, allyl or propargyl.

6. The use according to Claim 1, in which $n = 0$, $R_1 = R_2 = H$, $R_3 = R_4 = CH_3$; $n = 0$, $R_1 = R_2 = R_3 = H$, $R_4 = CH_2OH$ or $CH_2OCH_3$; $n = 0$, $R_1 = R_3 = H$, $R_2 + R_4 = CH_2CH_2CH_2CH_2$; or $n = 0$, $R_1 = R_2 = R_3 = H$, $R_4 = C_2H_5$.

7. The use according to any of Claims 1 to 6, in which $R_5$ is hydrogen.

8. The use according to Claim 1, in which $R_5$ is not hydrogen and $n = 0$, $R_1 = R_2 = R_3 = R_4 = H$ or $n = 0$, $R_1 = R_2 = R_3 = H$, $R_4 = CH_3$ or $C_2H_5$.

9. The use according to any of Claims 1 to 6 and 8, in which $R_5$ is a $C_{1-4}$ alkyl group.

10. The use according to Claim 9, in which $R_5$ is methyl or ethyl.

11. The use according to Claim 1, in which n = 0, $R_1$ = $R_2$ = $R_3$ = H, $R_4$ = H, $CH_3$ or $C_2H_5$, $R_5$ = $CH_3$; n = 0, $R_1$ = $R_3$ = H, $R_2$ = $R_4$ = $CH_3$, $R_5$ = H; or n = 0, $R_1$ = $R_2$ = $R_3$ = $R_5$ = H, $R_4$ = $C_2H_5$.

12. The use according to Claim 1, in which the compound of formula (II) is dl -, d - or l -1,2-bis(3,5-dioxopiperazin-1-yl)-butane.

13. The use according to Claim 1, in which the compound of formula (II) is 1,2-bis(2-methyl-3,5-dioxopiperazin-1-yl)-ethane, 1,2-bis(2-methyl-3,5-dioxopiperazin-1-yl)-propane, or 1,2-bis(2-methyl-3,5-dioxopiperazin-1-yl)-butane.

14. The use according to any of Claims 1 to 13, in which the compound is used in conjunction with an anthracycline drug.

15. The use according to Claim 14, in which the anthracycline drug is doxorubicin.

16. A pharmaceutical composition comprising a compound of formula (II):

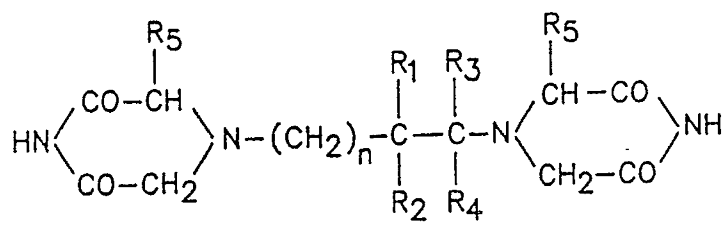

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of up to six carbon atoms, but with the provisos that (a) when n is 0 the combinations $R_1$ = $R_2$ = $R_3$ = $R_4$ = $R_5$ = H and $R_1$ = $R_2$ = $R_3$ = $R_5$ = H, $R_4$ = methyl are excluded, (b) when n is 1, the combination $R_1$ = $R_2$ = $R_3$ = $R_4$ = $R_5$ = H is excluded, (c) when n is 0, $R_1$, $R_3$ and $R_5$ are each hydrogen and each of $R_2$ and $R_4$ is other than hydrogen, the compound is in other than the meso or erythro configuration and (d) when n is 0, $R_1$ = $R_2$ = $R_3$ = $R_5$ = H and $R_4$ = $C_2H_5$ the compound is in other than the dl configuration, or a salt thereof formed with a physiologically acceptable inorganic or organic acid, together with a physiologically acceptable diluent or carrier excluding any liquid which is not sterile and pyrogen free.

17. A pharmaceutical composition comprising a compound of formula (II):

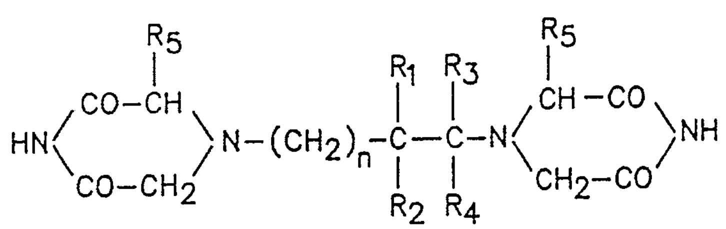

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1$ = $R_2$ = $R_3$ = $R_4$ = $R_5$ = H and $R_1$ = $R_2$ = $R_3$ = $R_5$ = H, $R_4$ = methyl are excluded, and (b) when n is 0, $R_1$, $R_3$ and $R_5$ are each hydrogen, and each of $R_2$ and $R_4$ is other than hydrogen the compound is in other than the meso or erythro configuration, or a salt thereof formed with a physiologically acceptable inorganic or organic acid, and an anthracycline drug, together with a physiologically acceptable diluent or carrier.

18. A pharmaceutical composition according to Claim 17, in which the anthracycline drug is doxorubicin.

19. A pharmaceutical composition comprising a compound of formula (II), according to Claim 12 or 13 or a salt thereof formed with a physiologically acceptable inorganic or organic acid, and doxorubicin, together with a physiologically acceptable diluent or carrier.

20. A pharmaceutical composition according to any of Claims 16 to 19 in a form suitable for parenteral administration.

21. A compound of formula (II):

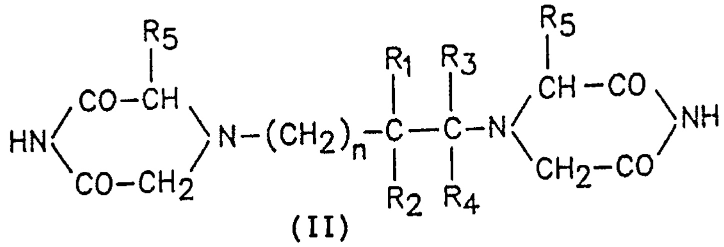

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, (b) when n is 0, $R_1$, $R_3$ and $R_5$ are each hydrogen, and each of $R_2$ and $R_4$ is other than hydrogen the compound is in other than the meso or erythro configuration, and (c) when n is 0, $R_1 = R_2 = R_3 = R_5 = H$ and $R_4 = C_2H_5$ the compound is in other than the dl configuration, or a salt thereof formed with a physiologically acceptable inorganic or organic acid, for use in therapy.

22. A compound according to Claim 21, which is as defined in any of Claims 2 to 13.

23. The use of a compound of formula (II):

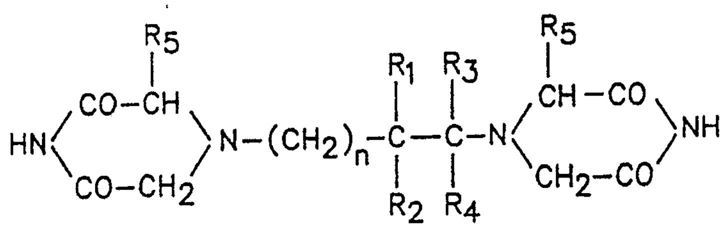

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, and (b) when n is 0, $R_1$, $R_3$ and $R_5$ are each hydrogen, and each of $R_2$ and $R_4$ is other than hydrogen the compound is in other than the meso or erythro configuration, or a salt thereof formed with a physiologically acceptable inorganic or organic acid, for use in the manufacture of a medicament for providing protection against the toxic effects of paracetamol.

24. The use of a compound of formula (II):

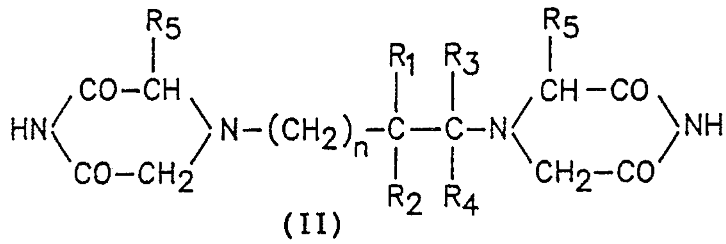

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or a $C_{1-6}$ alkoxy group, or one of $R_1$ and $R_2$ and one of $R_3$ and $R_4$ is hydrogen and the others are a trimethylene, tetramethylene or pentamethylene bridging group, and $R_5$ is hydrogen or an unsubstituted acyclic aliphatic hydrocarbon group having a maximum of six carbon atoms, but with the provisos that (a) when n is 0 the combinations $R_1 = R_2 = R_3 = R_4 = R_5 = H$ and $R_1 = R_2 = R_3 = R_5 = H$, $R_4$ = methyl are excluded, and (b) when n is 0, $R_1$, $R_3$ and $R_5$ are each hydrogen, and each of $R_2$ and $R_4$ is other than hydrogen the compound is in other than the meso or erythro configuration, or a salt thereof formed with a physiologically acceptable inorganic or organic acid, for use in the manufacture of a medicament for providing protection against damage caused by free radicals.

25. The use according to Claim 23 or 24, in which the compound formula (II) is as defined in any of Claims 2 to 13.

26. 1,2-bis(3,5-Dioxopiperazin-1-yl) butane in a form in which amount of the compound in either the d or l configuration is greater than that in the l or d configuration, respectively, or salt thereof formed with a physiologically acceptable inorganic organic acid.

27. 1,2,-bis(2-methyl-3,5-dioxopiperazin-1-yl)-propane or 1,2-bis(2-methyl-3,5-dioxopiperazin-1-yl)butane, or a salt thereof formed with a physiologically acceptable inorganic or organic acid.

28. A method for the treatment of a patient in need of cardioprotection or in need of protection against the toxic effects of paracetamol or of free radicals which comprises administering to said patient a therapeutically effective amount of a compound of formula (II) as defined in any of Claims 1 to 13.